# EUROPÄISCHE PATENTANMELDUNG

(11) **EP 1 690 529 A1**
(43) Veröffentlichungstag der Anmeldung: **16.08.2006**
(21) Anmeldenummer: 05003181.4
(22) Anmeldetag: 15.02.2005
(51) Int. Cl.: A61K 9/20

(54) **Feste perorale Arzneiform zur Kontrazeption, die Dienogest und Ethinylestradiol enthält**

(71) Anmelder: Schering AG, 13342 Berlin (DE)
(72) Erfinder: Fricke, Sabine, 07749 Jena (DE); Gerecke, Hagen, 07743 Jena (DE); Ladwig, Ralf, 07747 Jena (DE); Buske, Alexander, 07745 Jena (DE); Räthe, Harald, 07318 Arnsgereuth (DE)
(74) Vertreter: Cramer, Eva-Maria

(57) **Zusammenfassung**

Eine feste perorale Arzneiform zur Empfängnisverhütung enthält als eine Wirkstoffkomponente 17α-Cyanomethyl-17-β-hydroxyestra-4,9-dien-3on (Dienogest) in einer täglichen Dosierung von gleich oder kleiner 2.0 mg und als andere Wirkstoffkomponente 17α-Ethinylestradiol (Ethinylestradiol) in einer täglichen Dosierung von kleiner 0.03 mg gemeinsam mit einem oder mehreren pharmazeutisch annehmbaren Trägern. Das Dienogest wird anteilig in zwei Phasen freigesetzt und das Ethinylestradiol wird zeitgleich mit der ersten Phase des Dienogest freigegeben.

## Beschreibung

Die Erfindung betrifft eine feste perorale Arzneiform zur Kontrazeption, welches 17α-Cyanomethyl-17-β-hydroxyestra-4,9-dien-3on (Dienogest) gleich oder kleiner 2.0 mg und 17α-Ethinylestradiol (Ethinylestradiol) kleiner 0.030 mg enthält und wobei das Dienogest in zwei Phasen freigegeben wird.

### Allgemeiner Stand der Technik

Orale empfängnisverhütende Mittel, bestehend aus einer Gestagenkomponente und einer Östrogenkomponente kamen erstmals in den frühen 60er Jahren auf den Markt. Drei wesentliche Eigenschaften charakterisieren das Profil der "Verhütungspille": kontrazeptive Sicherheit, eine sehr gute Zykluskontrolle sowie ein Minimum an Nebenwirkungen.
Seit Einführung hormoneller Kontrazeptiva ist die Forschung auf die Möglichkeit zur Schaffung von Arzneiformen gerichtet, die bei gleichbleibend guter kontrazeptiver Sicherheit und Zykluskontrolle unerwünschte Nebenwirkungen, wie beispielsweise arterielle und venöse Thrombosen und Beeinflussung des Kohlehydrat- und Fettstoffwechsels - hervorgerufen durch einen höheren Gehalt an Gestagen und Estrogen als zur Empfängnisverhütung notwendig - , reduziert. WO 98/004269 offenbart u. a. die orale Verabreichung einer Kombination von 250 µg - 4 mg Dienogest und 10 µg - 20 µg Ethinylestradiol zur Empfängnisverhütung. Um die wesentliche Verringerung des pro Zyklus verabreichten gesamten empfängnisverhütenden Steroids zu erreichen, während eine gute Zykluskontrolle beibehalten wird, verabreicht man die niedrig dosierte Gestagen/Östrogen-Kombination für 23 bis 25 Tage eines 28-tägigen Menstruationszyklus. Jedoch werden in der Patentschrift keine Ergebnisse und Angaben offenbart, die belegen, dass die erfinderische Idee auch zum Erfolg führt und welche Art der Freisetzung der Steroide angestrebt wird.
WO 01/015701 beansprucht eine pharmazeutische Zusammensetzung auch zur oralen Verabreichung von 21 Tagen eines 28-tägigen Menstruationszyklus, die Drospirenon und Ethinylestradiol u. a. auch niedrig dosiert enthält, wobei Drospirenon in mikronisierter Form vorliegt. Besonderes Augenmerk wird hierbei auf eine schnelle Freisetzung der Steroide gelegt.
EP 0 803 250 offenbart eine pharmazeutische Tablette, welche einen pharmakologisch wirkstoffreien Tablettenkern besitzt und deren äußerer Zuckerüberzug u.a. Dienogest und Ethinylestradiol enthalten kann und wobei deren angestrebte schnelle Freisetzung durch mikrokristalline Cellulose beeinflusst wird.
Es ist auch bekannt, dass bei niedrig dosierten oralen Kontrazeptiva unbedingt auf den täglich gleichen Zeitraum der Einnahme zu achten ist. Bei Nichteinhaltung dieses Zeitraumes wird die zur oralen Kontrazeption notwendig wirksame Wirkstoffkonzentration unterschritten und die orale Kontrazeption ist nicht mehr gewährleistet. Das bedeutet, dass die Anwenderin angehalten ist, ihren Einnahmezyklus sehr bewusst und mit großer Sorgfalt zu verfolgen.

### Detaillierte Offenbarung der Erfindung

Der Erfindung liegt die Aufgabe zugrunde, eine Möglichkeit zu schaffen, die eine Reduzierung der herkömmlichen Dienogest/Ethinylestradiol-Wirkstoffkombination für orale Kontrazeptiva realisiert und dabei eine kontrazeptive Wirksamkeit analog der konventionellen oralen Kontrazeptiva sichert.
Diese Aufgabe wird erfindungsgemäß durch eine feste perorale Arzneiform zur Kontrazeption, als Wirkstoffkombination Dienogest gleich oder kleiner 2.0 mg und Ethinylestradiol kleiner 0.030 mg enthaltend, gelöst, wobei das Dienogest anteilig in zwei Phasen freigegeben wird.

Ausführungsformen sind Arzneiformen, als Wirkstoffkombination enthaltend 1.5 mg bis 2 mg Dienogest und 0.015 mg bis 0.020 mg Ethinylestrad iol.
In der erfindungsgemäßen Arzneiform besitzt vorzugsweise das Dienogest eine schnelle in-vitro-Freisetzung innerhalb der ersten Phase und eine verzögerte in-vitro-Freisetzung innerhalb der zweiten Phase) und das Ethinylestradiol eine konventionelle schnelle in-vitro-Freisetzung.
Der in der zweiten Phase freigesetzte Anteil an Dienogest beträgt mindestens 5 %, vorzugsweise größer 30 % wie mit dem Dissolutionstest nach Ph.Eur. mittels Drehkörbchenapparatur unter Verwendung von 1000 ml Wasser mit 37 °C als Dissolutionmedium und einer Rührgeschwindigkeit von 50 U/Min bestimmt,
In der zweiten Phase kann 10 % bis 90 % der Dosis an Dienogest nach bis zu 180 Min bis 360 Min freigestzt werden.
In der ersten Phase erfolgt die in-vitro-Freisetzung der Dosis an Ethinylestradiol und des Anteils an der Dosis von Dienogest zu mindestens 75 % in maximal 45 Min, vorzugsweise zu 70 % in 30 Min wie mit vorbezeichneten Dissolutionstest bestimmt.
Eine Ausführungsformen ist eine Filmtablette mit einem wirkstoffhaltigen Filmüberzug ist, deren Tablettenkern den Gehalt an Dienogest der zweiten Phase und der Filmüberzug den Gehaltes an Dienogest der ersten Phase und den Gesamtgehalt an Ethinylestradiol enthält.
Es wurde gefunden, dass Ascorbinsäure Ethinylestradiol stabilisiert sobald sie dem wirkstoffhaltigen Filmüberzug beigefügt ist.
Dabei beträgt der Ascorbinsäureanteil 0.02 bis 1.0 %, vorzugsweise 0.025 bis 0.25 %.
Die Wirkstofffreisetzung des retardierten Anteils Dienogest kann durch eine Vielzahl von Retardierungsprinzipien gesteuert werden. Beispiele dieser Retardierungsprinzipien sind inerte Kunstoffmatrizes, Hydrokolloide, lonenaustauscher, Retardierte Umhüllungen, magensaftresistente Überzüge, Pelletmischungen, Mischungen von Minitabletten und/oder Granulaten, Mikrokapseln, osmotisch kontrollierte Systeme, erosionsgesteuerte Systeme, diffusionsgesteuerte Systeme und deren Kombinationen, fett- und wachshaltige Matrizes.
Die in den Beispiele gezeigten Tablettenformen sind Modelle. Andere Tablettenformen wie oblonge Tabletten sind denkbar, ebenso wie Tablettenformen, die in ihrer Ausprägung das Erosionsverhalten hydrophiler Matrizes beeinflussen.
Es wurde überraschenderweise gefunden, dass die teilweise verzögerte Freisetzung des Dienogest aus der Arzneiform die niedrige Dosierung der Wirkstoffkombination Dienogest/Ethinylestradiol mit gleicher kontrazeptiver Wirksamkeit wie die konventionellen oralen Kontrazeptiva, Dienogest und Ethinylestradiol enthaltend ermöglicht und eine Einnahmesicherheit gewährleistet wird ohne unbedingt auf den täglich gleichen Zeitraum der Einnahme achten zu müssen.
Bei geeigneten Ausführungsformen des erfindungsgemäßen pharmazeutischen Präparates kann die Anzahl der Tagesdosiseinheiten 21, 22, 23, 24 oder 25 Tagesdosiseinheiten betragen und die Anzahl
der wirkstofffreien Tagesdosiseinheiten beträgt dann 7, 6, 5, 4 oder 3 im 28-tägigen Menstruationszyklus.
Weitere Ausführungsformen, mit einer Anzahl an Tagesdosiseinheiten von 28 oder ein vielfaches von 28, beispielsweise 2 bis 3 mal 28 , die Dienogest gleich oder kleiner 2.0 mg und Ethinylestradiol kleiner 0.030 mg enthalten, sind möglich.
Auch andere Ausführungsformen mit Gestagenen, wie beispielsweise Levonorgestrel, Gestoden und weitere und/ oder Estradiolvalerat, die neben der Empfängnisverhütung auch zur Hormonsubstitution (auch sequentiell) geeignet sind, sind möglich.
Die in den Beispielen gezeigten Ausführungsformen lassen sich auch hinsichtlich der Dosierung variieren. Der Anteil an Dienogest der zweiten, retardierten Phase sollte vorzugsweise größer als 30 % der Dienogestdosis sein. Das schließt Ausführungen mit 35 %, 40 %, 45 %, 50 % oder 55 %, aber auch beispielsweise 70 %, 75 %, 80 % der Dienogestgesamtdosis ein. Die Zeit, in der der Anteil an Dienogest der zweiten, retardierten Phase vollständig freigesetzt wird, variiert in den Beispielen von 180 min bis 360 min. Durch Variationen der Beispiele lassen jedoch auch kürzere oder längere Freisetzungszeiten erzielen.

Durch die übliche Variationsbreite der Wirkstofffreisetzung bedingt, gibt man die Freisetzungszeit in ganzen Stunden an, beispielsweise 1 h, 2 h, 3 h, 4 h als 6 h, 7 h usw.
Es hat sich herausgestellt, dass die Wirkstoffkombination im erfindungsgemäßen pharmazeutischen Präparat neben der Empfängnisverhütung antiandrogene Eigenschaften besitzt und daher bei der Prophylaxe und Therapie von androgeninduzierten Störungen, insbesonders von Akne, verwendet werden kann.

### Kurzdarstellung der Zeichnungen

Die Erfindung wird unter Bezugnahme auf die folgenden Zeichnungen näher beschrieben, auf welchen
Fig. 1 eine Kurve ist, die das Freisetzungsprofil von Valette® mit 2 mg Dienogest und 0.030 mg Ethinylestradiol darstellen, hergestellt und gemessen nach Beispiel 1;
Fig. 2 eine Kurve ist, die die Freisetzungsprofile von Filmtabletten mit 2 mg Dienogest und 0.020 mg Ethinylestradiol darstellen, hergestellt und gemessen nach den Beispiel 2 und 3;

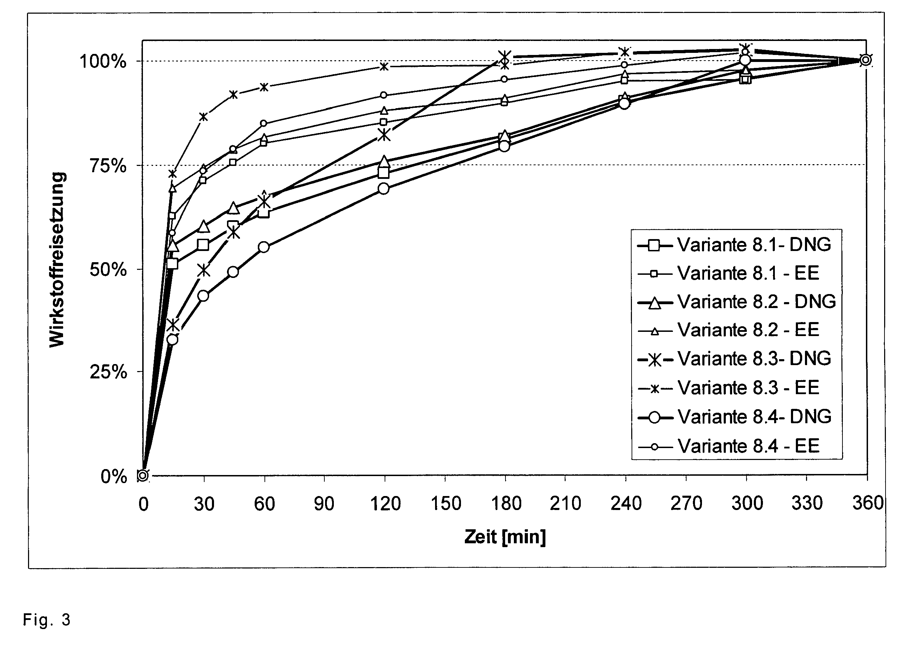

Fig. 3 eine Kurve ist, die die Freisetzungsprofile von Filmtabletten mit 1.5 mg Dienogest und 0.015 mg Ethinylestradiol darstellen, hergestellt und gemessen nach Beispiel 8;

### Beispiele

### Beispiel 1

### Beschreibung

Valette ist ein konventionelles Dragee zur oralen Kontrazeption, enthaltend 0.030 mg Ethinylestradiol und 2.0 mg Dienogest in einem

Tablettenkern, der mit einer zuckerhaltigen Hülle überzogen ist. Prüfung des Freisetzunasprofils
- Dissolutiontest nach Ph. Eur., 4. Ausgabe, Grundwerk 2002, 2.9.3, Blattrührerapparatur, 50 U/min, Dissolutionmedium 1000 ml Wasser
- Messung der freigesetzten Menge Dienogest und Ethinylestradiol mittels Hochdruckflüssigchromatographie

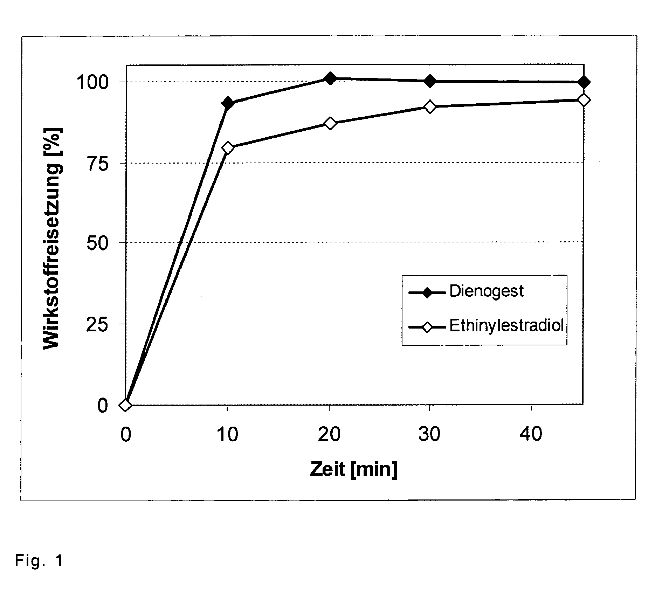

Figur 1 zeigt das typische Freisetzungsprofil von derartigen kontrazeptiven Kombinationen aus Gestagen und Estrogen zu erkennen. Ein derartiges Freisetzungsverhalten von mindestens 75 % der Wirkstoffdosis innerhalb von längstens 45 Min, vorzugsweise von 70 % in 30 Min wird schnell freisetzend genannt.

### Beispiel 2

2 mg Dienogest und 0.02 mg Ethinylestradiol, wobei 1 mg Dienogest verzögert und 1 mg Dienogest und 0.02 mg Ethinylestradiol schnell freigesetzt werden.

### Beschreibung

Das Beispiel beschreibt eine Filmtablette mit Matrixkern. Der Kern der Filmtablette enthält 1 mg Dienogest in einer hydrophilen Erosionsmatrix mit dem Grundbestandteil Metolose. Diese Matrix gibt den Wirkstoff Dienogest retardiert frei. Der Kern wurde mit einem schnell löslichen Film überzogen, der 1.0 mg Dienogest und 0.02 mg Ethinylestradiol enthält. Zum Lichtschutz wurde die Filmtablette mit einer weiteren schnell löslichen, Eisenoxidpigmente enthaltenden Farbschicht überzogen.

| Zusammensetzung | | | |
|---|---|---|---|
| Kern | | Filmhülle | |
| Granulat 1 | | Film 1 - wirkstoffhaltig | |
| Dienogest | 1.000 mg | Dienogest | 1.000 mg |
| Metolose 90SH-4000 | 7.500 mg | Ethinylestradiol | 0.020 mg |
| Laktose-Monohydrat | 21.000 mg | Methocel 5 | 2.250 mg |
| Maisstärke | 14.000 mg | Talkum | 0.450 mg |
| Povidon K25 (10 % in Ethanol) | 1.500 mg | Titandioxid | 0.280 mg |
| Granulat 2 | | Film 2 - Farbschicht | |
| Laktose-Monohydrat | 54.000 mg | Methocel 5 | 3.375 mg |
| Maisstärke | 27.100 mg | Talkum | 0.675 mg |
| Maltodextrin (25 % in Wasser) | 6.900 mg | Titandioxid | 1.875 mg |
| äußere Phase | | Eisenoxid, rot | 0.075 mg |
| Carboxymethylstärke-Natrium | 1.500 mg | | |
| Magnesiumstearat | 1.500 mg | | |

### Herstellung

- Granulat 1: Povidon in Ethanol lösen, mit dieser Lösung die anderen Substanzen im Wirbelschichtgranulator granulieren
- Granulat 2: Maltodextrin in Wasser lösen, mit dieser Lösung die anderen Substanzen im Wirbelschichtgranulator granulieren
- Granulat 1, Granulat 2 und äußere Phase im Containermischer mischen
- Mischung tablettieren (136 mg, oblonge Stempel 4,0x10,0 mm, Wölbung 4,5 mm)
- Film1: Tabletten mit Suspension aus Substanzen in Wasser überziehen im Trommelcoater
- Film 2: Filmtabletten mit Suspension aus Substanzen in Wasser überziehen im Trommelcoater

### Prüfung des Freisetzungsprofils

- Dissolutiontest nach Ph. Eur., 4. Ausgabe, Grundwerk 2002, 2.9.3, Drehkörbchenapparatur, 50 U/min, Dissolutionmedium 1000 ml Wasser
- Messung der freigesetzten Menge Dienogest und Ethinylestradiol mittels Hochdruckflüssigchromatographie

### Beispiel 3

2 mg Dienogest und 0.02 mg Ethinylestradiol, wobei 1 mg Dienogest verzögert und 1 mg Dienogest und 0.02 mg Ethinylestradiol schnell freigesetzt werden.

### Beschreibung

Das Beispiel beschreibt eine Filmtablette mit Matrixkern. Der Kern der Filmtablette enthält 1 mg Dienogest in einer hydrophilen Erosionsmatrix mit dem Grundbestandteil Metolose. Diese Matrix gibt den Wirkstoff Dienogest retardiert frei. Zur Vermeidung von Interaktionen zwischen retardierendem Kern und dem wirkstoffhaltigen Film wurde der Kern mit einer Sperrschicht überzogen, ehe der wirkstoffhaltige Film aufgebracht wurde. Der wirkstoffhaltige Film enthält 1.0 mg Dienogest und 0.02 mg Ethinylestradiol sowie Eisenoxidpigmente als Lichtschutz.

| Zusammensetzung | | | |
|---|---|---|---|
| Kern | | Filmhülle | |
| Granulat | | Sperrschicht | |
| Dienogest | 1.000 mg | Opadry AMB white® | 7.000 mg |
| Metolose 90SH-4000 | 7.500 mg | bestehend aus: | |
| Laktose-Monohydrat | 31.000 mg | Polyvinylalcohol - part. hydolisiert | |
| Maisstärke | 24.000 mg | Titandioxid | |
| Povidon K25 (10 % in Ethanol) | 2.000 mg | Soyalecithin | |
| | | Xanthan | |

| äußere Phase | | wirkstoffhaltiger Film | |
|---|---|---|---|
| Tablettose | 21.000 mg | | |
| Avicel PH 102 | 16.200 mg | Dienogest | 1.000 mg |
| Magnesiumstearat | 1.300 mg | Ethinylestradiol | 0.020 mg |
| | | Methocel 5 | 2.250 mg |
| | | Talkum | 0.430 mg |
| | | Titandioxid | 0.280 mg |
| | | Eisenoxid, rot | 0.020 mg |

### Herstellung

- Granulat: Povidon in Ethanol lösen, mit dieser Lösung die anderen Substanzen im Wirbelschichtgranulator granulieren
- Granulat 1 und äußere Phase im Containermischer mischen
- Mischung tablettieren (104 mg, oblonge Stempel 4,0x10,0 mm, Wölbung 4,5 mm)
- Sperrschicht: Tabletten mit Suspension aus Substanzen in Wasser überziehen im Trommelcoater
- wirkstoffhaltiger Film: Filmtabletten mit Suspension aus Substanzen in Wasser überziehen im Trommelcoater

### Prüfung des Freisetzunpsprofils

- Dissolutiontest nach Ph. Eur., 4. Ausgabe, Grundwerk 2002, 2.9.3, Drehkörbchenapparatur, 50 U/min, Dissolutionmedium 1000 ml Wasser
- Messung der freigesetzten Menge Dienogest und Ethinylestradiol mittels Hochdruckflüssigchromatographie

Die Filmtabletten der Beispiele 2 und 3 unterscheiden sich im Aufbau der Filmhülle. Der wirkstoffhaltige Film löst sich schnell auf und setzt die Ethinylestradioldosis und den Anteil der Dienogestdosis schnell frei. Die Farbschicht in Beispiel 2 löst sich ebenfalls schnell auf. Sie sorgt für Gefälligkeit und Lichtschutz der Filmtablette. Die Sperrschicht in Beispiel 3 unterbindet Interaktionen zwischen wirkstoffhaltigem Film und dem Kern und löst sich langsamer als der wirkstoffhaltige Film auf. Beide Filmtabletten weisen einen Matrixkern auf, bestehend aus einer hydrophilen Erosionsmatrix. Diese Erosionsmatrix setzt den retardierten Anteil der Dienogestdosis langsam frei.

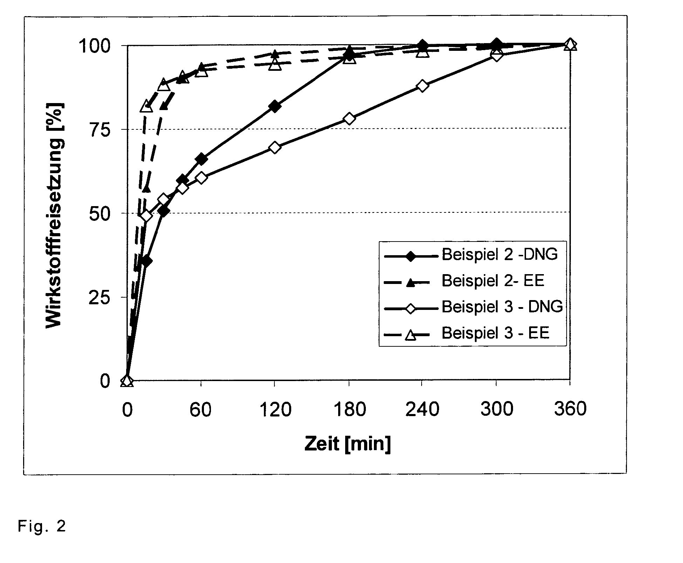

Figur 2 zeigt die gemessenen Freisetzungsprofile der Beispiele 2 und 3 gezeigt. Die Filmtabletten beider Beispiele setzen ca. 80 % der Ethinylestradioldosis innerhalb von 45 min sowie ca. 50 % der Dienogestdosis innerhalb von 45 min frei. Der restliche Anteil der Dienogestdosis wird in der Filmtablette aus Beispiel 2 innerhalb von 360 min und bei Beispiel 3 innerhalb von 180 min freigesetzt.

### Beispiel 4

Im Beispiel 4 wird die Freisetzung des retardierten Anteils Dienogest mit einer lipohilen Matrix gesteuert.

### Beschreibung

Das Beispiel beschreibt eine Tablette enthaltend 2 mg Dienogest und 0.020 mg. Ethinylestradiol mit einem lipophilen Retardierungsprinzip. 1 mg Dienogest wird in eine lipophile Matrix durch Versprühen eingebettet. Der schnell freisetzende Anteil Dienogest sowie das Ethinylestradiol werden dieser Matrix zugemischt.

| Zusammensetzung | |
|---|---|
| Dienogest (in ethanolischer Lösung) | 1.00 mg |
| Cetylstearylakohol (in ethanolischer Lösung) | 9.00 mg |
| Laktose-Monohydrat | 57.98 mg |
| Maisstärke | 10.00 mg |
| Dienogest | 1.00 mg |
| Ethinylestradiol | 0.02 mg |
| Maltodextrin (20%ige Lösung in Wasser) | 9.00 mg |
| Na-Carboxymethylstärke | 1.00 mg |
| Magnesiumstearat | 1.00 mg |

### Herstellung

- retardierter Anteil Dienogest und Cetylstearylakohol in Ethanol von 50 °C lösen
- Dienogest/Cetylstearylkohol-Lösung im Wirbelschichtgranulat auf Laktose und Maisstärke aufsprühen und trocknen
- Ethinylestradiol, 2. Hälfte der Dosis Dienogest trocken untermischen
- Maltodextrin in Wasser lösen
- Mischung in der Wirbelschicht mit Maltodextrinlösung granulieren
- Na-Carboxymethylstärke und Magnesiumstearat untermischen
- zu Tabletten Durchmesser 5,5 mm mit Masse 90 mg tablettieren

### Beispiel 5

Im Beispiel 5 wird die Freisetzung des Dienogest durch Verwendung unterschiedlicher Korngrößen gesteuert.

### Beschreibung

Das Beispiel beschreibt eine Tablette, bei der Dienogest mit verschiedenen Kornfraktionen eingesetzt wird. Die gezielte Einstellung der Partikelgröße erfolgt mittels fraktionierter Kristallisation.

| Zusammensetzung | |
|---|---|
| Dienogest (mittlere Korngröße 3 µm) | 0.667 mg |
| Dienogest (mittlere Korngröße 180 µm) | 0.667 mg |
| Dienogest (mittlere Korngröße 270 µm) | 0.667 mg |
| Ethinylestradiol | 0.02 mg |
| Laktose-Monohydrat | 47.18 mg |
| Maisstärke | 24.00 mg |
| Maltodextrin (20%ige Lösung in Wasser) | 9.00 mg |
| Magnesiumstearat | 0.80 mg |

### Herstellung:

- Granulieren der Substanzen mit Maltodextrinlösung
- Trocknen des Granulates
- Zumischen von Magnesiumstearat
- Mischung tablettieren (Durchmesser 5,5 mm mit Masse 80 mg)

### Beispiel 6

Bei der Verringerung der Ethinylestradioldosis stellt sich ein chemisches Problem. Die zunehmende Verdünnung des Wirkstoffs in der Arzneiform beschleunigt dessen chemische Zersetzung im Verlaufe der Lagerung, möglicherweise bereits während der Herstellung der Arzneiform. Überraschenderweise zeigte sich Ascorbinsäure als wirksamer Stabilisator für Ethinylestradiol. Beispiel 6 zeigt die Wirksamkeit der Ascorbinsäure als Stabilisator in Beispielrezepturen.

### Beschreibung

Das Beispiel beschreibt den stabilisierenden Effekt von Ascorbinsäure auf Ethinylestradiol anhand von Beispielrezepturen im Stresstest.

| Zusammensetzung | | | | | |
|---|---|---|---|---|---|
| Variante | 6.1 | 6.2 | 6.3 | 6.4 | 6.5 |
| Ascorbinsäure, in Bindemittellösung | - | 0.02 mg | 0.20 mg | - | - |
| Ascorbinsäure, in Mischung | - | - | - | 0.02 mg | 0.20 mg |
| Dienogest | 2.00 mg | 2.00 mg | 2.00 mg | 2.00 mg | 2.00 mg |
| Ethinylestradiol | 0.02 mg | 0.02 mg | 0.02 mg | 0.02 mg | 0.02 mg |
| Laktose-Monohydrat | 47.18 mg | 47.16 mg | 46.98 mg | 47.16 mg | 46.98 mg |
| Maisstärke | 24.00 mg | 24.00 mg | 24.00 mg | 24.00 mg | 24.00 mg |
| Maltodextrin | 6.00 mg | 6.00 mg | 6.00 mg | 6.00 mg | 6.00 mg |
| Magnesiumstearat | 0.80 mg | 0.80 mg | 0.80 mg | 0.80 mg | 0.80 mg |

Herstellung
- Laktose, Maisstärke und Dienogest im Granulator mischen, mit einer Lösung von Ethinylestradiol in Ethanol besprühen und trocknen
- Mischung mit Bindemittellösung von Maltodextrin in Wasser granulieren, trocknen und mit Magnesiumstearat mischen
- Mischung zu Tabletten von 80 mg Masse und Durchmesser 5,5 mm tablettieren

### Prüfung des Gehaltes im Stresstest

Die Tabletten werden im offenen Gefäß bei 60 °C und 80 % relativer Feuchte eingelagert. Nach Ablauf der Lagerzeit von 42 Tagen werden die Tabletten entnommen und untersucht. Die Messung des Gehaltes an Ethinylestradiol erfolgt mittels HPLC und wird auf den Gehalt des Startwertes bezogen.

| Variante | 6.1 | 6.2 | 6.3 | 6.4 | 6.5 |
|---|---|---|---|---|---|
| Ascorbinsäure, in Bindemittellösung | - | 0.02 mg | 0.20 mg | - | - |
| Ascorbinsäure, in Mischung | - | - | - | 0.02 mg | 0.20 mg |
| Gehalt nach 42 Tagen | 79.1 % | 90.1 % | 91.9 % | 91.6 % | 96.5 % |

### Beispiel 7

### Beschreibung

In diesem Beispiel wird die Stabilisierung des Ethinylestradiols in der wirkstoffhaltigen Schicht von Filmtabletten beschrieben.

| Zusammensetzung | |
|---|---|
| Kern | 104 mg |
| Dienogest | 0.750 mg |
| Metolose 90SH-4000 | 7.500 mg |
| Laktose-Monohydrat | 45.250 mg |
| Maisstärke | 10.000 mg |
| Povidon K25 (10 % in Wasser) | 2.000 mg |
| Tablettose | 30.000 mg |
| Avicel PH 102 | 7.200 mg |
| Magnesiumstearat | 1.300 mg |

| Sperrschicht | 6 mg |
|---|---|
| Eudragit RL 30 D (als Lacktrockensubstanz) | 3.500 mg |
| Macrogol 6000 | 0.700 mg |
| Talkum | 1.800 mg |

| wirkstoffhaltiger Film | 3 mg |
|---|---|
| Dienogest | 0.750 mg |
| Ethinylestradiol | 0.015 mg |
| Ascorbinsäure | 0.200 mg |
| Methocel 5 | 1.6875 mg |
| Talkum | 0.2375 mg |
| Titandioxid | 0.210 mg |

| Farbschicht | 3 mg |
|---|---|
| Methocel 5 | 1.500 mg |
| PEG 6000 | 0.300 mg |
| Talkum | 0.300 mg |
| Titandioxid | 0.850 mg |
| Eisenoxid, rot | 0.050 mg |

### Herstellung

- Dienogest, Metolose 90SH-4000, Laktose-Monohydrat und Maisstärke werden mit der wässrigen Povidon K25- bzw. Maltodextrinlösung granuliert
- dem getrockneten Granulat werden Tablettose, Avicel PH 102 und Magnesiumstearat zugemischt
- die Mischung wird tablettiert
- die Tabletten werden im Trommelcoater mit der entsprechenden Filmen überzogen

### Beispiel 8

### Beschreibung

Es werden vier Varianten von Filmtabletten der Gesamtdosis 1.5 mg Dienogest und 0.015 mg Ethinylestradiol beschrieben. Die Filmtabletten bestehen aus einem retardierenden Matrixkern und einer schnell löslichen Filmhülle sowie einer Farbschicht. In zwei Varianten wurde zusätzlich eine Sperrschicht zwischen Kern und wirkstoffhaltigem Film vorgesehen.
In den vier Varianten der Filmtablette wurde der retardierte Anteil der Dienogestdosis im Bereich von 33 % bis 66 % verändert. Die Rezeptur des Kerns wurde den gewünschten Freisetzungsprofilen angepasst.

| Zusammensetzung | | | | |
|---|---|---|---|---|
| Variante | 8.1 | 8.2 | 8.3 | 8.4 |
| Kern | 104 mg | 104 mg | 104 mg | 104 mg |
| Dienogest | 0.750 mg | 0.500 mg | 1.000 mg | 0.750 mg |
| Metolose 90SH-4000 | 7.500 mg | 7.500 mg | 7.500 mg | 9.000 mg |
| Laktose-Monohydrat | 45.250 mg | 39.500 mg | 45.000 mg | 29.750 mg |
| Maisstärke | 10.000 mg | 10.000 mg | 10.000 mg | 24.000 mg |
| Povidon K25 (10 % in | 2.000 mg | - | 2.000 mg | 2.000 mg |
| Wasser) | - | 8.000 mg | - | - |
| Maltodextrin (25 % in | 30.000 mg | 30.000 mg | 30.000 mg | 21.000 mg |
| Wasser) | 7.200 mg | 7.200 mg | 7.200 mg | 16.200 mg |
| Tablettose | 1.300 mg | 1.300 mg | 1.300 mg | 1.300 mg |
| Avicel PH 102 | | | | |
| Magnesiumstearat | | | | |

| Sperrschicht | 6 mg | 6 mg | - | - |
|---|---|---|---|---|
| Eudragit RL 30 D | 3.500 mg | 3.500 mg | | |
| (als Lacktrockensubstanz) | 0.700 mg | 0.700 mg | | |
| Macrogol 6000 | 1.800 mg | 1.800 mg | | |
| Talkum | | | | |

| wirkstoffhaltiger Film | 3 mg | 3 mg | 3 mg | 3 mg |
|---|---|---|---|---|
| Dienogest | 0.750 mg | 1.000 mg | 0.500 mg | 0.750 mg |
| Ethinylestradiol | 0.015 mg | 0.015 mg | 0.015 mg | 0.015 mg |
| Methocel 5 | 1.6875 mg | 1.4987 mg | 1.8848 mg | 1.6875 mg |
| Talkum | 0.3375 mg | 0.2998 mg | 0.3700 mg | 0.3375 mg |
| Titandioxid | 0.210 mg | 0.18650 mg | 0.2302 mg | 0.210 mg |

| Farbschicht | 3 mg | 3 mg | 3 mg | 3 mg |
|---|---|---|---|---|
| Methocel 5 | 1.500 mg | 1.500 mg | 1.500 mg | 1.500 mg |
| PEG 6000 | 0.300 mg | 0.300 mg | 0.300 mg | 0.300 mg |
| Talkum | 0.300 mg | 0.300 mg | 0.300 mg | 0.300 mg |
| Titandioxid | 0.850 mg | 0.850 mg | 0.850 mg | 0.850 mg |
| Eisenoxid, rot | 0.050 mg | 0.050 mg | 0.050 mg | 0.050 mg |

### Herstellung

- Dienogest, Metolose 90SH-4000, Laktose-Monohydrat und Maisstärke werden mit der wässrigen Povidon K25- bzw. Maltodextrinlösung granuliert
- dem getrockneten Granulat werden Tablettose, Avicel PH 102 und Magnesiumstearat zugemischt
- die Mischung wird tablettiert
- die Tabletten werden im Trommelcoater mit der entsprechenden Filmen überzogen

### Prüfung des Freisetzungsprofils

- Dissolutiontest nach Ph. Eur., 4. Ausgabe, Grundwerk 2002, 2.9.3, Drehkörbchenapparatur, 50 U/min, Dissolutionmedium 1000ml Wasser
- Messung der freigesetzten Menge Dienogest und Ethinylestradiol mittels Hochdruckflüssigchromatographie

## Patentansprüche

1. Feste perorale Arzneiform zur Kontrazeption, als Wirkstoffkombination Dienogest gleich oder kleiner 2.0 mg und Ethinylestradiol kleiner 0.030 mg enthaltend, wobei das Dienogest anteilig in zwei Phasen freigegeben wird.

2. Arzneiform nach Anspruch 1, wobei die Wirkstoffkombination 1.5 mg bis 2.0 mg Dienogest und 0.015 mg bis 0.020 mg Ethinylestradiol enthält.

3. Arzneiform nach einem der Ansprüche 1 bis 2, wobei der freigegebene Anteil an Dienogest in der zweiten Phase mindestens 5 %, vorzugsweise größer 30 % beträgt wie mit dem Dissolutionstest unter Verwendung von Wasser mit 37 °C als Dissolutionmedium und 50 U/Min als Rührgeschwindigkeit bestimmt.

4. Arzneiform nach einem der Ansprüche 1 bis 4, wobei die Arzneiform eine Filmtablette mit einem wirkstoffhaltigen Filmüberzug ist, deren Tablettenkern den Gehalt an Dienogest der zweiten Phase und der Filmüberzug den Gehaltes an Dienogest der ersten Phase und den Gesamtgehalt an Ethinylestradiol enthält.

5. Arzneiform nach einem der Ansprüche 1 bis 5, wobei dem wirkstoffhaltigen Filmüberzug Ascorbinsäure als Stabilisator des Ethinylestradiol beigefügt ist.

6. Arzneiform nach Anspruch 7, wobei der Ascorbinsäureanteil 0.02 bis 1.0 %, vorzugsweise 0.025 bis 0.25 % beträgt.

7. Arzneiform nach einem der Ansprüche 1 bis 6, wobei die Anzahl der Tagesdosiseinheiten, die die Kombination aus Dienogest und Ethinylestradiol enthalten, 21, 22, 23, 24 oder 25 beträgt und die Anzahl der Tagesdosiseinheiten, die keinen Wirkstoff enthalten, 7, 6, 5, 4 oder 3 beträgt.

## Geänderte Patentansprüche

### Geänderte Patentansprüche gemäss Regel 86(2) EPÜ.

**1.** Feste perorale Arzneiform zur Kontrazeption, als Wirkstoffkombination Dienogest gleich oder kleiner 2.0 mg und Ethinylestradiol kleiner 0.030 mg enthaltend, wobei die Arzneiform eine Filmtablette darstellt, bestehend aus einem Tablettenkern mit einem retardierend freizusetzenden anteiligem Gehalt zum Gesamtgehalt an Dienogest und einem Filmüberzug mit einem nicht retardierend (schnell) freizusetzenden anteiligem Gehalt zum Gesamtgehalt an Dienogest und einem nicht retardierend (schnell) freizusetzenden Gesamtgehalt an Ethinylestradiol.

**2.** Arzneiform nach Anspruch 1, wobei die Wirkstoffkombination 1.5 mg bis 2.0 mg Dienogest und 0.015 mg bis 0.020 mg Ethinylestradiol enthält.

**3.** Arzneiform nach einem der Ansprüche 1 bis 2, wobei mindestens 10 %, vorzugsweise 30 % an Dienogest retardiert nach größer 30 Minuten aus dem Tablettenkern heraus gelöst werden, wie mit dem Dissolutionstest unter Verwendung von Wasser mit 37 °C als Dissolutionmedium und 50 U/Min als Rührgeschwindigkeit bestimmt.

**4.** Arzneiform nach einem der Ansprüche 1 bis 4, wobei dem wirkstoffhaltigen Filmüberzug Ascorbinsäure als Stabilisator des Ethinylestradiol beigefügt ist.

**5.** Arzneiform nach Anspruch 5, wobei der Ascorbinsäureanteil 0.02 bis 1.0 %, vorzugsweise 0.025 bis 0.25 % beträgt.

**6.** Arzneiform nach einem der Ansprüche 1 bis 6, wobei die Anzahl der Tagesdosiseinheiten, die die Kombination aus Dienogest und Ethinylestradiol enthalten, 21, 22, 23, 24 oder 25 beträgt und die Anzahl der Tagesdosiseinheiten, die keinen Wirkstoff enthalten, 7, 6, 5, 4 oder 3 beträgt.
